(19) Europäisches Patentamt / European Patent Office / Office européen des brevets

(11) **EP 4 129 462 A1**

(12) **EUROPEAN PATENT APPLICATION**
published in accordance with Art. 153(4) EPC

(43) Date of publication:
**08.02.2023 Bulletin 2023/06**

(21) Application number: **21781704.8**

(22) Date of filing: **22.02.2021**

(51) International Patent Classification (IPC):
**B01J 19/00** (1980.01)        **G16C 20/10** (2019.01)

(52) Cooperative Patent Classification (CPC):
**B01J 19/00; G16C 20/10**

(86) International application number:
**PCT/JP2021/006568**

(87) International publication number:
**WO 2021/199788 (07.10.2021 Gazette 2021/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.04.2020 JP 2020067796**

(71) Applicant: **Daikin Industries, Ltd.**
**Osaka-shi, Osaka 530-0001 (JP)**

(72) Inventors:
• **TAKAKUWA, Tatsuya**
**Osaka-shi, Osaka 530-8323 (JP)**

• **NOGUCHI, Atsushi**
**Osaka-shi, Osaka 530-8323 (JP)**
• **HASUMOTO, Yuuta**
**Osaka-shi, Osaka 530-8323 (JP)**
• **YOSHIZAKI, Satoru**
**Osaka-shi, Osaka 530-8323 (JP)**
• **MURATA, Hiroyuki**
**Osaka-shi, Osaka 530-8323 (JP)**
• **IWASAKI, Tsuneyoshi**
**Osaka-shi, Osaka 530-8323 (JP)**

(74) Representative: **Hoffmann Eitle**
**Patent- und Rechtsanwälte PartmbB**
**Arabellastraße 30**
**81925 München (DE)**

(54) **ANALYSIS METHOD THAT ANALYSES CHEMICAL REACTIONS FROM STARTING MATERIALS, ANALYSIS DEVICE, ANALYSIS SYSTEM, AND ANALYSIS PROGRAM**

(57) This disclosure relates to an analysis method for analyzing a chemical reaction from one or more starting materials, comprising
a preparation step S1 of preparing a reaction network diagram indicating the one or more starting materials, at least part of one or more intermediate products and one or more final products generated from the chemical reaction, and reaction pathways of the chemical reaction, and
a prediction step S2 of predicting the reaction rate in each reaction pathway using an artificial intelligence algorithm.

**(Cont. next page)**

EP 4 129 462 A1

Fig.1

```
                    ┌──────────┐
                    │   Start  │
                    └──────────┘
                         │
          ┌───────────────────────────┐
          ║      Preparation step      ║──── S1
          └───────────────────────────┘
                         │
          ┌───────────────────────────┐
          ║      Prediction step       ║──── S2
          └───────────────────────────┘
                         │
                         ▼ ◄──────────────────────┐
          ┌───────────────────────────┐           │
          │      Performing step       │──── S3     │
          └───────────────────────────┘           │
                         │                         │
          ┌───────────────────────────┐           │
          │       Updating step        │──── S4     │
          └───────────────────────────┘           │
                         │                         │
          ┌───────────────────────────┐           │
          │     Reaction condition     │──── S5     │
          │        search step         │           │
          └───────────────────────────┘           │
                         │            S6           │
                       ◇◇◇◇◇                       │
                    Have the          NO           │
              steps been sufficiently ─────────────┘
                    repeated?
                       ◇◇◇◇◇
                         │ YES
                    ┌──────────┐
                    │   End    │
                    └──────────┘
```

**Description**

Technical Field

The present disclosure relates to an analysis method, analysis apparatus, analysis system, and analysis program, each analyzing chemical reaction from a starting materials.

Background Art

In order to efficiently obtain a desired product from a starting material by chemical reaction, researchers have employed a method in which experiments on chemical reactions are repeatedly conducted by changing reaction conditions based on researchers' own empirical rules by using a reaction device to search for more efficient reaction conditions. Such a method requires researchers to determine reaction conditions on their own, and further, it is time-consuming because the operation of the reaction device and the analysis of data are conducted by a person.

In contrast, PTL 1 discloses repeating a cycle comprising analyzing a chemical reaction of a subset of compounds selected from a library of compounds based on specific selection criteria and selecting a subset of compounds from a library of compounds based on the selection criteria generated from the above analysis, thereby generating chemical entities with defined physical, chemical, and/or bioactive properties. PTL 2 discloses a reaction mechanism generation method comprising the step of performing a molecular dynamics calculation at each time step of atoms that constitute each molecule in a reaction system, the step, performed when a chemical reaction has occurred in the reaction system before and after the time step, of identifying a reacting molecule and a product molecule that contributed to the chemical reaction, the step of constructing, based on atomic relationships between the reacting molecule and the product molecule, an elementary reaction structured from the reacting molecule and the product molecule having the relationship, and the step of calculating a reaction rate constant for the constructed elementary reaction.

Citation List

Patent Literature

    PTL 1: JP2001-507675A
    PTL 2: WO2016/133002

Summary of Invention

Technical Problem

However, in PTL 1, the selection criteria of a subset of compounds do not include the concept of the reaction rate. In PTL 2, although the reaction rate constants for individual chemical reactions are calculated, divergent reaction pathways of the chemical reactions from initial molecules are not considered. Accordingly, PTL 1 and 2 have difficulty in searching for reaction conditions to efficiently obtain a desired product.

The present disclosure has been accomplished in order to solve the above problems, and an object of the present disclosure is to efficiently search for good reaction conditions for chemical reaction.

Solution to Problem

In order to achieve the above object, the analysis method according to one embodiment of the present disclosure is a method for analyzing a chemical reaction from one or more starting materials, comprising preparing a reaction network diagram indicating the one or more starting materials, at least part of one or more intermediate products and one or more final products generated from the chemical reaction, and reaction pathways of the chemical reaction, and predicting the reaction rate in each reaction pathway using an artificial intelligence algorithm.

In the analysis method, predicting the reaction rate preferably comprises calculating the rate constant indicating the reaction rate, and modifying the rate constant with the artificial intelligence algorithm.

In the analysis method, in calculating the rate constant, the rate constant can be preferably calculated based on the Arrhenius equation.

In the analysis method, the artificial intelligence algorithm is preferably a gradient algorithm, a genetic algorithm, or a combination thereof.

It is further preferable that the analysis method comprise performing the chemical reaction more than once under different reaction conditions using a reaction device, and updating the predicted reaction rate based on the results of the performed chemical reactions.

The analysis method according to one embodiment of the present disclosure is a method for analyzing a chemical reaction from one or more starting materials, comprising performing the chemical reaction more than once under different reaction conditions using a reaction device, and updating, based on the results of the performed chemical reactions, the reaction rate in each reaction pathway of the reaction network diagram indicating the one or more starting materials, at least part of one or more intermediate products and one or more final products generated from the chemical reaction, and reaction pathways of the chemical reaction.

In the analysis method, it is preferable to update the reaction rate by an automatic differential method in updating the reaction rate.

It is preferable that the analysis method further comprise searching for an optimal reaction condition based on the different reaction conditions and the results of the performed chemical reactions corresponding to respective reaction conditions.

In the analysis method, it is preferable to search for the optimal reaction condition by Bayesian optimization in searching for the optimal reaction condition.

The analysis apparatus of the present disclosure is an apparatus for analyzing a chemical reaction from one or more starting materials, and the apparatus comprises a preparation unit that prepares a reaction network diagram indicating the one or more starting materials, at least part of one or more intermediate products and one or more final products generated from the chemical reaction, and reaction pathways of the chemical reaction, and a prediction unit that predicts the reaction rate in each reaction pathway using an artificial intelligence algorithm.

The analysis system according to one embodiment of the present disclosure comprises the analysis apparatus of the present disclosure and a reaction device that performs the chemical reaction more than once under different reaction conditions, wherein the analysis apparatus further comprises an updating unit that updates the predicted reaction rate based on the results obtained by the reaction device.

The analysis program according to one embodiment of the present disclosure is a program for analyzing a chemical reaction from one or more starting materials, wherein the analysis program causes a computer to perform preparing a reaction network diagram indicating the one or more starting materials, at least part of one or more intermediate products and one or more final products generated from the chemical reaction, and reaction pathways of the chemical reaction, and predicting the reaction rate in each reaction pathway using an artificial intelligence algorithm.

Advantageous Effects of Invention

According to the present disclosure, excellent reaction conditions for chemical reaction can be efficiently searched for.

Brief Description of Drawings

Fig. 1 is a flowchart showing the process of an analysis method according to an embodiment of the present disclosure.
Fig. 2 is a functional block diagram showing the schematic structure of an analysis system according to an embodiment of the present disclosure.
Fig. 3 is an example of a reaction network diagram.
Fig. 4 is a flowchart showing the details of a preparation step.
Fig. 5 is a flowchart showing the details of a prediction step.
Fig. 6 is an example of a reaction network diagram.

Fig. 7 is an example of a reaction network diagram.
Fig. 8 is an example of a reaction network diagram.
Fig. 9 is an illustrative diagram showing an example of Bayesian optimization.
Fig. 10 is an example of a reaction network diagram.
Fig. 11 is an illustrative diagram showing an example of Bayesian optimization.

Description of Embodiments

Preferable embodiments of the present disclosure are described below with reference to the attached drawings. The present disclosure is not limited to the following embodiments.

Summary of analysis method

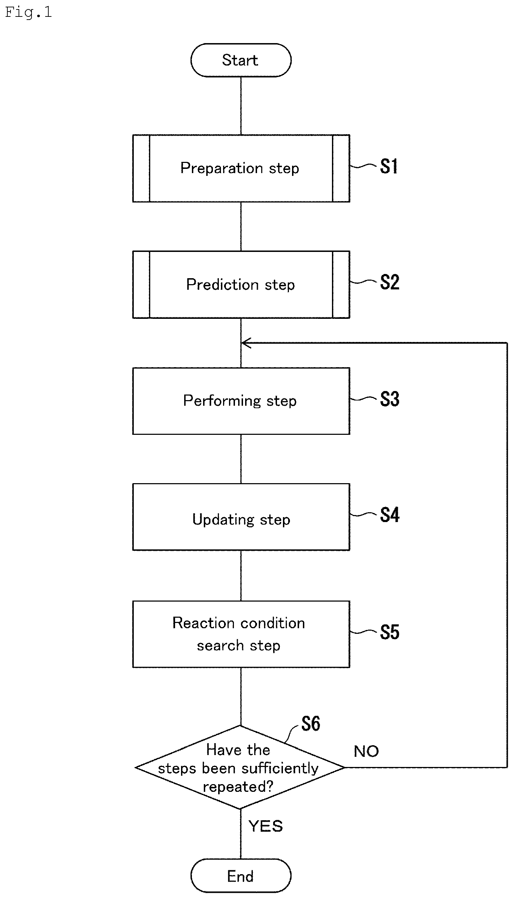

Fig. 1 is a flowchart showing the process of an analysis method according to the present embodiment. The analysis method according to the present embodiment is a method for analyzing a chemical reaction from one or more starting materials, comprising:

a preparation step S1 of preparing a reaction network diagram indicating the one or more starting materials, at least part of one or more intermediate products and one or more final products generated from the chemical reaction, and reaction pathways of the chemical reaction;
a prediction step S2 of predicting the reaction rate in each reaction pathway using an artificial intelligence algorithm;
a performing step S3 of performing the chemical reaction more than once under different reaction conditions using a reaction device;
an updating step S4 of updating the predicted reaction rate based on the results of the performing step S3; and
a reaction condition search step S5 of searching for an optimal reaction condition based on the different reaction conditions and the results of the performing step S3 corresponding to each reaction condition.

The chemical reaction may be a degradation reaction, synthesis reaction, or polymerization reaction; however, it is more preferably a degradation reaction. Examples of the degradation reaction include degradation of a pharmaceutical agent (degradation relating to metabolism in the body), ozone (layer) depletion by CFCs, degradation and detoxification of NOx in exhaust gas, analysis of degradation mechanisms of chemical products, and etching processing.

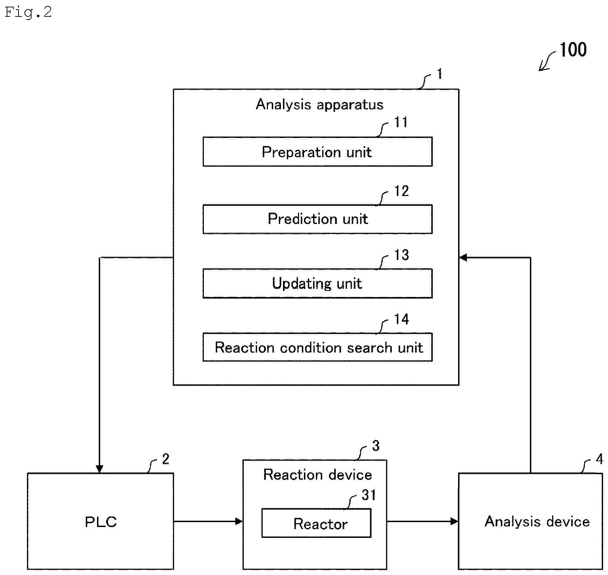

Steps S1 to S5 can be performed by the analysis system 100 of the present embodiment. The details of processing of steps S1 to S5 are described below with reference to Figs. 2 to 11.

Structure of analysis system

Fig. 2 is a functional block diagram showing the schematic structure of the analysis system 100. The analysis system 100 includes an analysis apparatus 1, a PLC 2, a reaction device 3, and an analysis device 4.

The analysis apparatus 1 is an apparatus for analyzing a chemical reaction from a starting material. The hardware configuration of the analysis apparatus 1 is not limited, and the analysis apparatus 1 can be formed of a general computer, a portable computer such as a tablet or smartphone, or a quantum computer.

The analysis apparatus 1 includes, as functional blocks, a preparation unit 11, a prediction unit 12, an updating unit 13, and a reaction condition search unit 14. When the analysis apparatus 1 is formed of a general computer, the units 11 to 14 are functioned when the GPU or CPU in the analysis apparatus 1 reads out the analysis program of this embodiment into the main memory to perform the analysis program. The analysis program may be downloaded to the analysis apparatus 1 via a communication network, such as the internet, or the analysis program may be recorded on a non-transient computerreadable recording medium such as a CD-ROM, and installed in the analysis apparatus 1 via the recording medium. The functions of units 11 to 14 are described below.

The PLC 2 is a control device for controlling the reaction device 3. The analysis apparatus 1 may have a function of PLC 2.

The reaction device 3 is a device for performing a chemical reaction such as a decomposition reaction or an additional reaction. The reaction device 3 comprises a reactor 31 for storing a chemical material, and allows a chemical reaction to consecutively proceed in the reactor 31 under the predetermined reaction conditions (e.g., temperature and residence time).

The analysis device 4 is a device that analyzes the components of a material in the reactor 31. The reaction device 3 may have a function of the analysis device 4.

Details of analysis method

The following section describes the details of processing when each of the steps S1 to S5 in the method for analyzing a chemical reaction from a starting material, which is shown in Fig. 1, is performed by the analysis system 100.

Preparation step

The preparation step S1 is performed by the preparation unit 11 in the analysis apparatus 1. The preparation unit 11 has a function of preparing a reaction network diagram indicating the one or more starting materials, at least part of one or more intermediate products and one or more final products generated from the chemical reaction, and reaction pathways of the chemical reaction.

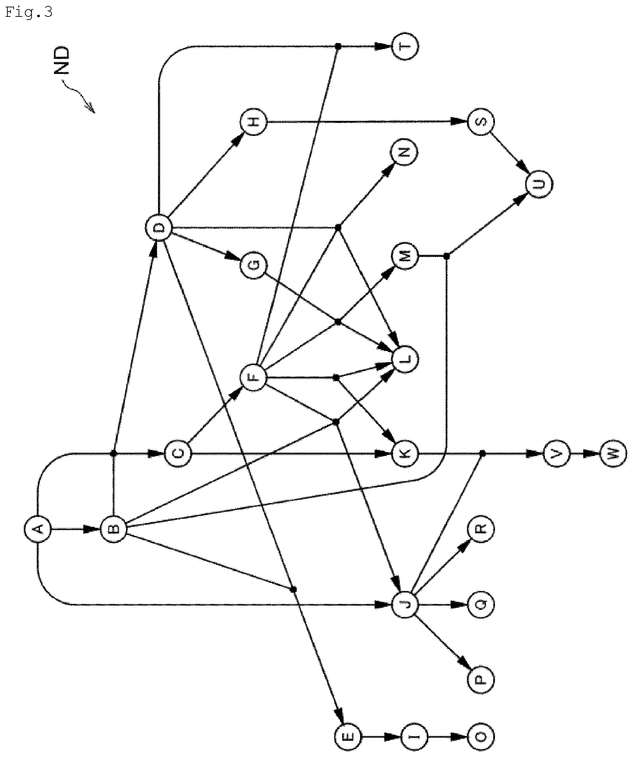

Fig. 3 is an example of a reaction network diagram ND. The reaction network diagram ND is a drawing in which a chemical reaction from a starting material is modeled, which shows a starting material A, intermediate products B to V, and a final product W produced by the chemical reaction, and reaction pathways of the chemical reaction. The intermediate products B to V and the final product W are part of the intermediate and final products resulting from the chemical reaction of the starting material A. The desired chemical material is contained in the intermediate products B to V and the final product W.

The starting material A, intermediate products B to V, and final product W can be gases, liquids, or solids, and are preferably gases. The starting material A, intermediate products B to V, and final product W may be low-molecular-weight compounds or high-molecular-weight compounds, and are preferably low-molecular-weight compounds. The number of intermediate products B to V and final product W, and the number of reaction pathways are not limited; however, they are determined by considering the calculation amount in the prediction step S2, updating step S4, and reaction condition search step S5 described below. The reaction network diagram ND may not include the final product.

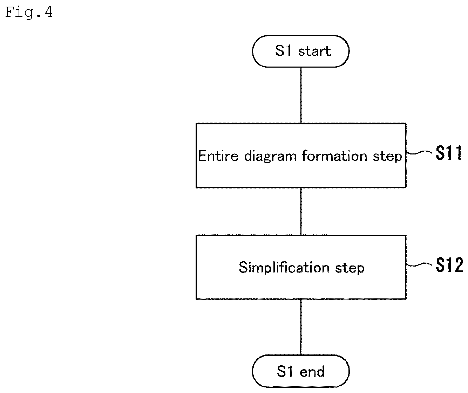

Fig. 4 is a flowchart showing the details of the preparation step S1. The preparation step S1 includes an entire diagram formation step S11 and a simplification step S12.

In the entire diagram formation step S11, when the starting material is determined by the user, the preparation unit 11 forms an exhaustive reaction network diagram (entire diagram) indicating the starting material(s), (almost) all of the intermediate product(s) and final product(s) resulting from the chemical reaction, and (almost) all of the reaction pathways of the chemical reaction. The entire diagram can be automatically formed according to a predefined reaction pattern. For example, if the chemical reaction is a degradation reaction, one or more intermediate products produced from the starting material(s) are determined according to the possible pattern of material(s) desorbed from the starting material(s). Similarly, intermediate products are additionally determined according to the possible pattern of material(s) desorbed from the intermediate product(s). This process is repeated to form the entire diagram.

The entire diagram does not necessarily include all of the intermediate products and final products. Similarly, the entire diagram does not necessarily include all the reaction pathways of the chemical reaction. The number of reaction pathways in the entire diagram is not limited. It is, for example, about 5000.

Although the entire diagram shows (almost) all of the reaction pathways, the use of the entire diagram in the prediction step S2, updating step S4, and reaction condition search step S5 results in a huge amount of calculation. Accordingly, in this embodiment, the simplification step S12 is performed.

In the simplification step S12, the preparation unit 11 simplifies the entire diagram to form the reaction network diagram ND shown in Fig. 3. The method of simplifying the entire diagram is not limited. For example, a reaction network diagram ND can be formed by performing a convolution operation on the entire diagram.

Thus, the preparation step S1 is completed.

Prediction step

The prediction step S2 is performed by the prediction unit 12 in the analysis apparatus 1. The prediction unit 12 has a function of predicting the reaction rate in each reaction pathway using an artificial intelligence algorithm. The artificial intelligence algorithm is not limited, and is preferably a gradient algorithm, a genetic algorithm, or a combination thereof.

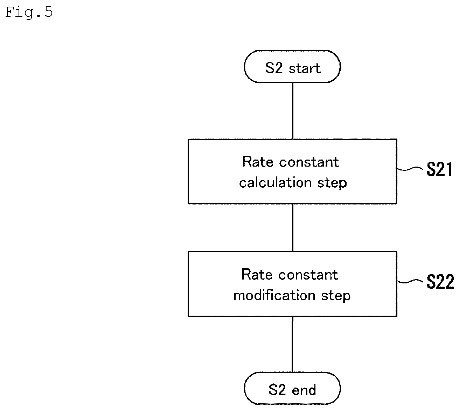

Fig. 5 is a flowchart showing the details of the prediction step S2. The prediction step S2 includes a rate constant calculation step S21 and a rate constant modification step S22.

In the rate constant calculation step S21, the prediction unit 12 calculates the rate constant showing the reaction rate in each reaction pathway. In this embodiment, the prediction unit 12 calculates the rate constant based on the Arrhenius equation. In the Arrhenius equation, the rate constant k is represented by the following formula:

$$k = A \exp\left(-\frac{E_a}{RT}\right)$$

wherein A is a frequency factor, Ea is an activation energy per mol, R is a gas constant, and T is an absolute temperature. Since the rate constant k varies according to the temperature and residence time in the chemical reaction, in the rate constant calculation step S21, the prediction unit 12 calculates the rate constants $k_{1-0}$ to $k_{n-0}$ (wherein n is the number of reaction pathways in the reaction network diagram ND) in individual reaction pathways under reaction conditions of temperature $T_0$ and residence time $t_0$ as the initial values of the rate constants. The temperature $T_0$ and residence time $t_0$ may be given by the user or randomly given by the prediction unit 12.

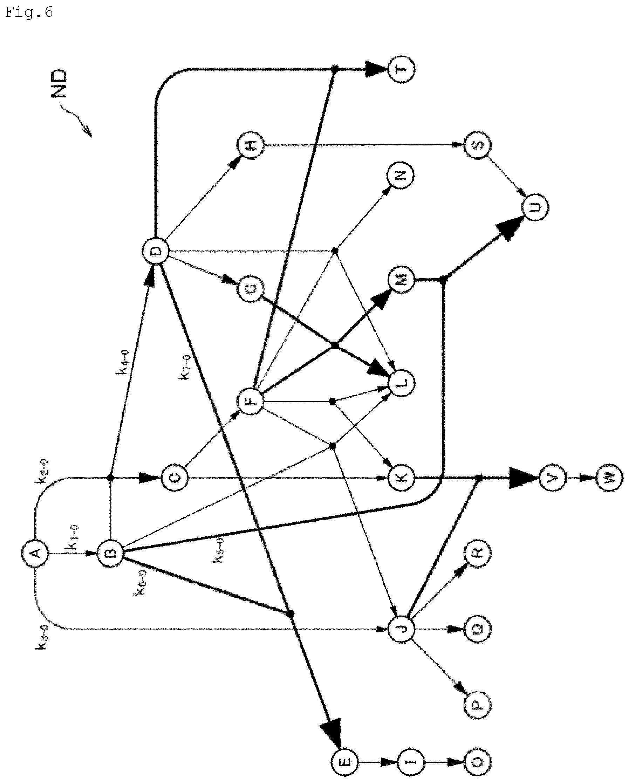

Fig. 6 is the reaction network diagram ND with initial values for rate constants. To avoid complication, only the rate constants $k_{1-0}$ to $k_{7-0}$ for the seven pathways are shown in Fig. 6. In order to intuitively understand the reaction rate in each pathway, the thickness of the line indicating the pathway in the reaction network diagram ND corresponds to the magnitude of the rate constant.

In the rate constant modification step S22, the prediction unit 12 modifies the rate constant by an artificial intelligence algorithm. In this embodiment, the rate constants $k_{1-0}$ to $k_{n-0}$ are modified by changing reaction conditions (temperature and residence time) using a gradient algorithm, a genetic algorithm, or a combination thereof as an artificial intelligence algorithm so that the value of the evaluation function (e.g., target chemical material yield and cost) increases. Thereby, reaction conditions under which the evaluation function is maximized are searched for, and the rate constants are converged to rate constants $k_{1-1}$ to $k_{n-1}$ in which the evaluation function is maximized in theory. The rate constants $k_{1-1}$ to $k_{n-1}$ are reaction rates predicted in the prediction step S2. The reaction conditions in this case are temperature $T_1$ and residence time $t_1$. The reaction conditions are not limited to temperature and residence time, and it may be, for example, reaction time, reaction pressure, raw material concentration, or raw material introduction flow rate.

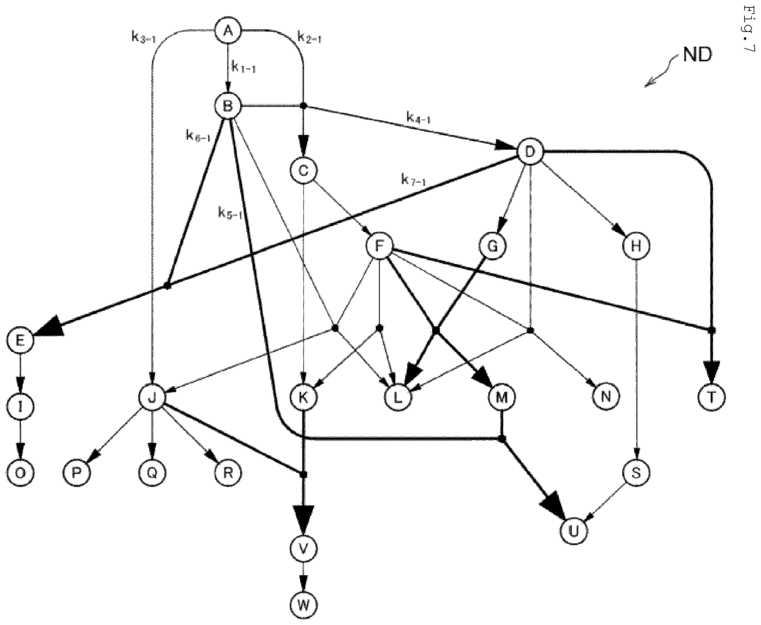

Fig. 7 is a reaction network diagram ND with theoretical optimum values of rate constants. To avoid complication, in Fig. 7, only rate constants $k_{1-1}$ to $k_{7-1}$ of the seven pathways are shown.

It is more preferable to use a genetic algorithm or an algorithm involving the combination of a gradient algorithm and a genetic algorithm because the rate constant may fall into a local solution if the gradient algorithm alone is used.

Thus, the prediction step S2 is completed.

Performing step, reaction condition search step, and updating step

In the performing step S3, the chemical reaction is performed more than once using the reaction device 3 under different reaction conditions. As described below, since the performing step S3 to the reaction condition search step S5 are performed more than once, in the single performing step S2, the chemical reaction is performed once under specific reaction conditions.

In this embodiment, the starting material A is subjected to chemical reaction in the reactor 31 of the reaction device 3 by changing reaction conditions within the range of temperature $T_2$ to $T_3$ and residence time $t_2$ to $t_3$. In this embodiment, the temperatures $T_2$ and $T_3$ satisfy $T_2<T_3$ and $T_1=(T_2+T_3)/2$; however, the temperatures $T_2$ and $T_3$ are not limited as long as they satisfy $T_2<T_1<T_3$, and are the temperatures at which the reaction device 3 can be operated. Similarly, the residence times $t_2$ and $t_3$ in this embodiment satisfy $t_2<t_3$ and $t_1=(t_2+t_3)/2$; however, the residence times $t_2$ and $t_3$ are not limited as long as they satisfy $t_2<t_1<t_3$. The reaction conditions of the chemical reaction are determined by the reaction condition search unit 14, which is described below; however, the reaction conditions of the chemical reaction may be set by the user if the reaction condition search step S5 is omitted, as described below. The PLC 2 controls the reaction device 3 according to the determined reaction conditions.

When the chemical reaction in the reaction device 3 is completed, the analysis device 4 analyzes the concentration of each chemical material in the reactor 31, and the yield of the target compound. Furthermore, the analysis device 4 provides feedback on the analyzed concentration and yield to the analysis apparatus 1 as the results of the performing step.

Subsequently, in the updating step S4, the updating unit 13 updates the reaction rates predicted in the prediction step S2, based on the results of the performing step S3. In this embodiment, the updating unit 13 updates the predicted reaction rate by an automatic differential method. Specifically, the updating unit 13 updates rate constants $k_{1-1}$ to $k_{n-1}$ to rate constants $k_{1-2}$ to $k_{n-2}$ in such a manner that the error is minimized by comparing the concentration obtained by the analysis device 4 and output results of the yield of the target compound with the concentration of a compound output from the reaction network diagram with the reaction rate group obtained in the immediately preceding prediction step S2 and output results of the yield of the target compound.

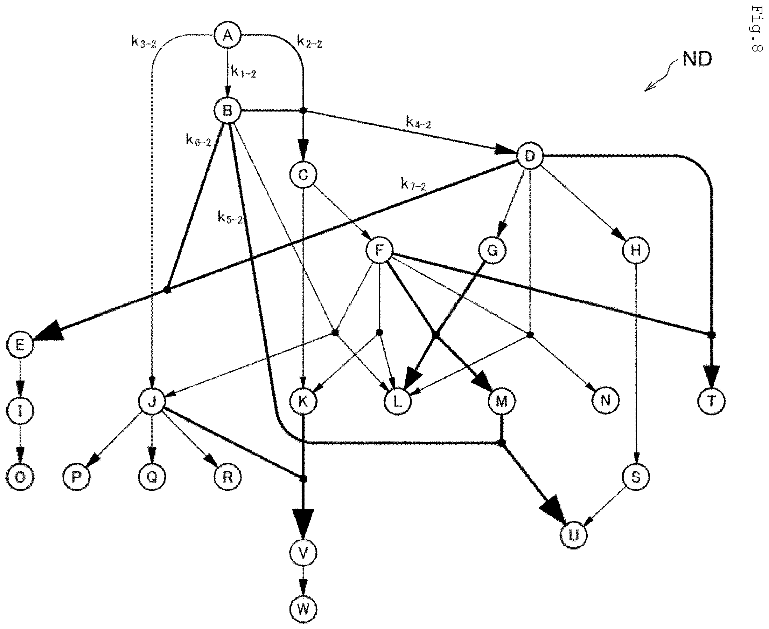

Fig. 8 is the reaction network diagram ND in which the rate constant is updated once. To avoid complication, in Fig. 8, only rate constants $k_{1-2}$ to $k_{7-2}$ of the seven pathways are shown.

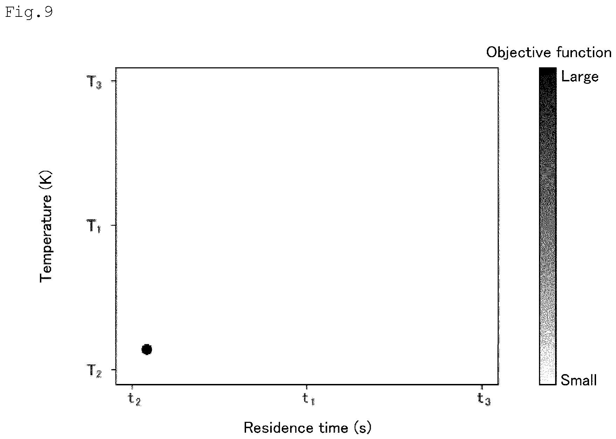

In the subsequent reaction condition search step S5, the reaction condition search unit 14 searches for optimal reaction conditions based on the reaction conditions and results of the performing step S3. In this embodiment, the reaction condition search unit 14 searches for the optimal reaction conditions by Bayesian optimization. In the first reaction condition search step S5, the reaction condition search unit 14 records reaction conditions and the results in the first performing step S3, as shown in Fig. 9. In Fig. 9, the shading of the circle corresponds to the degree of the objective function, with a smaller (lighter) objective function value meaning a higher yield (excellent results). The reaction condition search unit 14 then determines reaction conditions for the subsequent performing step S3 based on optimization theory.

The number of times in which the performing step S3 to the reaction condition search step S5 are repeated is not limited; however, it is preferable that the steps be repeated (m times) until the rate constant is sufficiently converged in the updating step S4, and an appropriate reaction condition can be searched for (no possibility of falling into a local solution) in the reaction condition search step S5 (YES in step S6).

For example, in the i-th (1<i<m) performing step S3, the PLC 2 controls the reaction device 3 according to the reaction conditions determined by the reaction condition search unit 14 in the (i-1)-th reaction condition search step S5 to perform a chemical reaction. In the i-th updating step S4, the updating unit 13 updates the rate constant in the reaction network diagram ND based on the results of the first performing step S3 to i-th performing step S3. The more times the updating step S4 is performed, the smaller the latitude of the rate constant becomes, and so the rate constant is converged to a predetermined value. In the i-th reaction condition search step S5, the reaction condition search unit 14 records the reaction conditions and results in the i-th performing step S3, and determines reaction conditions for the (i+1)-th performing step S3 based on the optimization theory.

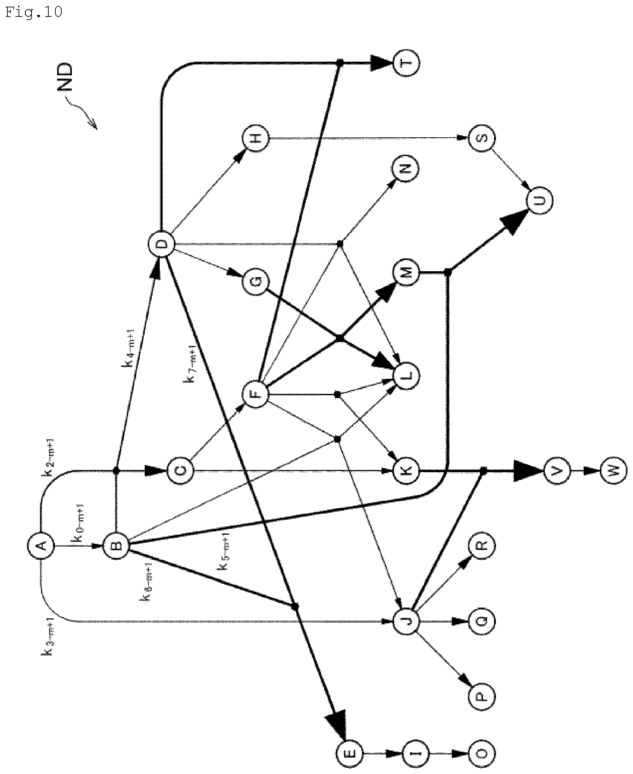

Fig. 10 is a reaction network diagram ND after completion of the m-th updating step S4. In Fig. 10, of the rate constants $k_{1-m+1}$ to $k_{n-m+1}$ in all of the pathways, only $k_{1-m+1}$ to $k_{7-m+1}$ are shown. The rate constants $k_{1-m+1}$ to $k_{7-m+1}$ can be regarded as the true rate constants in the reaction network diagram ND.

Based on the reaction network diagram ND in Fig. 10, a reaction pathway showing a high yield can be easily found. By-products generated by chemical reaction are often not considered in conventional reaction pathway searches; however, since the reaction network diagram ND includes by-products, it is expected to find reaction pathways that have been overlooked by users' own empirical rules or existing theories.

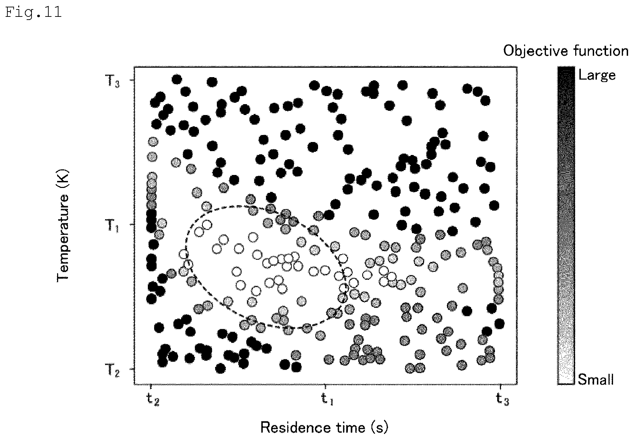

Fig. 11 is an illustrative diagram showing an example of Bayesian optimization after optimization (after completion of the m-th reaction condition search step S5). In Fig. 11, the number of plots showing reaction conditions is m, which suggests that the optimal reaction conditions exist within the dashed circle. Based on this, optimal or near-optimal reaction conditions can be easily found.

As described above, in this embodiment, the reaction network diagram is prepared in the preparation step S1, and the reaction rates in the reaction network diagram are predicted in the prediction step S2 with an artificial intelligence algorithm such as a gradient method algorithm. Since the reaction network diagram is formed based on chemical knowledge, and the reaction rates are predicted with an artificial intelligence algorithm considering the reaction pathways shown in the reaction network diagram, excellent reaction conditions for chemical reaction can be efficiently searched for. In contrast, PTL 1 does not consider the reaction rate of the chemical reaction. In PTL 2, a reaction network diagram showing reaction pathways of chemical reaction generated from a starting material (initial molecule) is not used, nor does it disclose the prediction of the reaction rate with an artificial intelligence algorithm considering the reaction pathways.

Furthermore, in the performing step S3 to the reaction condition search step S5, since reaction conditions are inductively searched for by Bayesian optimization or the like based on the results of the chemical reaction using the reaction device, more excellent reaction conditions can be searched for in a shorter time. This reduces the work required for reaction condition search, and results in highly efficient search.

Additional remarks

The embodiments are described above; however, it will be understood that various changes in forms and details can be made without departing from the spirit and scope of the claims.

For example, in the above embodiments, the reaction condition search step S5 is performed after the updating step S4; however, the updating step S4 may be performed after the reaction condition search step S5. Alternatively, the updating step S4 and the reaction condition search step S5 may be simultaneously performed.

The reaction condition search step S5 may be omitted. In such a case, the performing step S3 and the updating step S4 may both be repeated once under different reaction conditions, or the performing step S3 may be performed beforehand multiple times under different reaction conditions, followed by the updating step S4.

The preparation step S1 and the prediction step S2, or the preparation step S1 alone may be omitted. In such a case, the user may, for example, prepare a reaction network diagram based on the user's own empirical rules.

The performing step S3 to the reaction condition search step S5 may be omitted, and reaction pathways may be searched for based on the reaction network diagram ND obtained in the prediction step S2. The reaction rates predicted in the prediction step S2 are theoretical reaction rates predicted with an artificial intelligence algorithm such as a gradient method algorithm; however, it is possible to find near-optimal reaction pathways as long as a huge error, such as falling into a local solution, does not occur.

In the above embodiment, a gradient algorithm, genetic algorithm, or combination thereof was used as the artificial intelligence algorithm in the prediction step S2; however, the artificial intelligence algorithm in the present disclosure is not limited to these. Artificial intelligence algorithms, such as the Gauss-Newton method and EM algorithm, can also be used in the prediction step S2.

In the above embodiment, in the reaction condition search step S5, the optimal reaction conditions are searched for by Bayesian optimization; however, the method of searching for optimal reaction conditions is not limited to this. In the reaction condition search step S5, a method such as a genetic algorithm, ant colony optimization, and reinforcement learning can also be used.

In the above embodiment, the reaction conditions to be searched for are temperature and residence time; however, the type and number of reaction conditions are not limited. For example, the reaction conditions may include the partial pressure of the raw material.

Industrial Applicability

The present disclosure can be applied for reaction condition search of chemical reaction for producing various chemical materials.

Description of Numerals

1: Analysis apparatus
11: Preparation unit
12: Prediction unit
13: Updating unit
14: Reaction condition search unit
2: PLC
3: Reaction device
31: Reactor
4: Analysis device
100: Analysis system
A: Starting material
B to V: Intermediate product
W: Final product
ND: Reaction network diagram
S1: Preparation step
S11: Entire diagram formation step
S12: Simplification step
S2: Prediction step
S21: Rate constant calculation step
S22: Rate constant modification step
S3: Performing step
S4 Updating step
S5: Reaction condition search step

**Claims**

1. An analysis method for analyzing a chemical reaction from one or more starting materials, comprising

   preparing a reaction network diagram indicating the one or more starting materials, at least part of one or more intermediate products and one or more final products generated from the chemical reaction, and reaction pathways of the chemical reaction, and
   predicting the reaction rate in each reaction pathway using an artificial intelligence algorithm.

2. The analysis method according to claim 1, wherein predicting the reaction rate comprises calculating the rate constant indicating the reaction rate, and modifying the rate constant with the artificial intelligence algorithm.

3. The analysis method according to claim 2, wherein in calculating the rate constant, the rate constant is calculated based on the Arrhenius equation.

4. The analysis method according to any one of claims 1 to 3, wherein the artificial intelligence algorithm is a gradient algorithm, a genetic algorithm, or a combination thereof.

5. The analysis method according to any one of claims 1 to 4, further comprising

   performing the chemical reaction more than once under different reaction conditions using a reaction device, and
   updating the predicted reaction rate based on the results of the performed chemical reactions.

6. An analysis method for analyzing a chemical reaction from one or more starting materials, comprising

   performing the chemical reaction more than once under different reaction conditions using a reaction device, and
   updating, based on the results of the performed chemical reactions, the reaction rate in each reaction pathway of the reaction network diagram indicating the one or more starting materials, at least part of one or more intermediate products and one or more final products generated from the chemical reaction, and reaction pathways of the chemical reaction.

7. The analysis method according to claim 5 or 6, wherein in updating the reaction rate, the reaction rate is updated by an automatic differential method.

8. The analysis method according to any one of claims 5 to 7, further comprising searching for an optimal reaction condition based on the different reaction conditions and the results of the performed chemical reactions corresponding to respective reaction conditions.

9. The analysis method according to claim 8, wherein in searching for the optimal reaction condition, the optimal reaction condition is searched for by Bayesian optimization.

10. An analysis apparatus for analyzing a chemical reaction from one or more starting materials, the apparatus comprising

    a preparation unit that prepares a reaction network diagram indicating the one or more starting materials, at least part of one or more intermediate products and one or more final products generated from the chemical reaction, and reaction pathways of the chemical reaction, and
    a prediction unit that predicts the reaction rate in each reaction pathway using an artificial intelligence algorithm.

11. An analysis system comprising the analysis apparatus according to Claim 10 and a reaction device that performs the chemical reaction more than once under different reaction conditions, wherein the analysis apparatus further comprises an updating unit that updates the predicted reaction rate based on the results obtained by the reaction device.

12. An analysis program for analyzing a chemical reaction from one or more starting materials, wherein the analysis program causes a computer to perform preparing a reaction network diagram indicating the one or more starting materials, at least part of one or more intermediate products and one or more final products generated from the chemical reaction, and reaction pathways of the chemical reaction, and predicting the reaction rate in each reaction pathway using an artificial intelligence algorithm.

Fig.1

```
                    ┌──────────┐
                    │  Start   │
                    └──────────┘
                         │
              ┌──────────────────────┐
              ║  Preparation step    ║ ── S1
              └──────────────────────┘
                         │
              ┌──────────────────────┐
              ║  Prediction step     ║ ── S2
              └──────────────────────┘
                         │
                         ▼◄───────────────────────┐
              ┌──────────────────────┐            │
              │  Performing step     │ ── S3       │
              └──────────────────────┘            │
                         │                        │
              ┌──────────────────────┐            │
              │  Updating step       │ ── S4       │
              └──────────────────────┘            │
                         │                        │
              ┌──────────────────────┐            │
              │  Reaction condition  │ ── S5       │
              │  search step         │            │
              └──────────────────────┘            │
                         │                        │
                      S6 ╱╲                        │
                       ╱    ╲                      │
                     ╱ Have the ╲  NO              │
                   ╱ steps been    ╲───────────────┘
                    ╲ sufficiently ╱
                     ╲ repeated?  ╱
                       ╲        ╱
                         ╲    ╱
                          YES
                           │
                    ┌──────────┐
                    │   End    │
                    └──────────┘
```

Fig.2

Fig.3

ND

Fig.4

S1 start

Entire diagram formation step — S11

Simplification step — S12

S1 end

Fig.5

```
        ╭──────────────╮
        │   S2 start    │
        ╰──────────────╯
               │
    ┌──────────────────────┐
    │    Rate constant      │ ～S21
    │   calculation step    │
    └──────────────────────┘
               │
    ┌──────────────────────┐
    │    Rate constant      │ ～S22
    │  modification step    │
    └──────────────────────┘
               │
        ╭──────────────╮
        │    S2 end      │
        ╰──────────────╯
```

Fig.6

Fig.7

Fig.8

Fig.9

Fig.10

Fig.11

| | INTERNATIONAL SEARCH REPORT | International application No. |
| --- | --- | --- |
| | | PCT/JP2021/006568 |

**A. CLASSIFICATION OF SUBJECT MATTER**
B01J 19/00(2006.01)i; G16C 20/10(2019.01)i
FI: G16C20/10; B01J19/00 321

According to International Patent Classification (IPC) or to both national classification and IPC

**B. FIELDS SEARCHED**

Minimum documentation searched (classification system followed by classification symbols)
G16C10/00-99/00; B01J14/00-19/32

Documentation searched other than minimum documentation to the extent that such documents are included in the fields searched
| | |
| --- | --- |
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2005-179199 A (TOYOTA MOTOR CORP.) 07 July 2005 (2005-07-07) paragraphs [0054], [0098], [0102], etc. | 1–12 |
| Y | JP 7-037821 A (HITACHI, LTD.) 07 February 1995 (1995-02-07) paragraphs [0009], [0015], [0019], etc. | 1–12 |

☐ Further documents are listed in the continuation of Box C.      ☒ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 26 April 2021 (26.04.2021) | 11 May 2021 (11.05.2021) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

## INTERNATIONAL SEARCH REPORT
Information on patent family members

| International application No. |
| --- |
| PCT/JP2021/006568 |

| Patent Documents referred in the Report | Publication Date | Patent Family | Publication Date |
| --- | --- | --- | --- |
| JP 2005-179199 A | 07 Jul. 2005 | (Family: none) | |
| JP 7-037821 A | 07 Feb. 1995 | (Family: none) | |

Form PCT/ISA/210 (patent family annex) (January 2015)

**REFERENCES CITED IN THE DESCRIPTION**

**Patent documents cited in the description**

- JP 2001507675 A

- WO 2016133002 A